# EUROPEAN PATENT APPLICATION

(11) **EP 1 181 933 A2**
(43) Date of publication of application: **27.02.2002**
(21) Application number: 01305039.8
(22) Date of filing: 08.06.2001
(51) Int. Cl.: A61K 31/444, A61K 31/55, A61K 31/438, A61K 31/437, A61P 3/00

(54) **Use of growth hormone secretagogues for stimulating or increasing appetite**

(30) Priority: 29.06.2000 US 214979 P
(71) Applicant: Pfizer Products Inc., Groton, Connecticut 06340 (US)
(72) Inventor: Friedman, Hylar Lewis, Pfizer Global Res. & Devel., Groton, Connecticut 06340 (US); Gardner, Mark James, Pfizer Global Res. & Devel., Groton, Connecticut 06340 (US); Landschulz, William H, Pfizer Global Res. & Devel., Groton, Connecticut 06340 (US); Pan, Lydia Codetta, Pfizer Global Res. & Devel., Groton, Connecticut 06340 (US)
(74) Representative: Stuart, Peter Graham

(57) **Abstract**

This invention is directed to methods for increasing or stimulating appetite in a patient which comprises administering certain growth hormone secretagogues, prodrugs thereof or pharmaceutically acceptable salts of said secretagogues or said prodrugs. More preferably, the present invention provides such methods wherein the growth hormone secretagogue is a compound of Formula I: 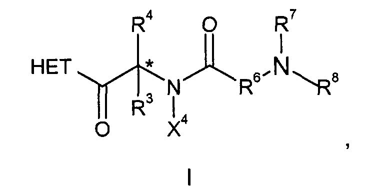 a prodrug thereof or a pharmaceutically acceptable salt of said secretagogue or said prodrug.

## Description

### FIELD OF THE INVENTION

The present invention provides methods of using certain growth hormone secretagogues, prodrugs thereof and pharmaceutically acceptable salts of said secretagogues and said prodrugs, for stimulating or increasing appetite in patients. More specifically, the present invention provides such methods wherein the growth hormone secretagogues are compounds of Formula I below. In addition, the present invention provides methods of treating eating disorders, such as anorexia nervosa, using certain growth hormone secretagogues, prodrugs thereof and pharmaceutically acceptable salts of said secretagogues and said prodrugs.

### BACKGROUND OF THE INVENTION

Aging is often accompanied by nutritional and metabolic decrements characterized by poor appetite and difficulty in maintaining body weight, particularly lean body mass. Thus, many older individuals suffer from eating disorders or malnutrition.

Chronically ill patients and patients suffering from acute diseases or conditions may have poor appetites due to their disease or condition or the treatment therefor. For example, patients undergoing cancer chemotherapy, patients who are immunocompromised due to, for example, AIDS or patients who are immunosuppressed due to, for example, organ transplants, commonly experience decreased appetite. Also, postoperative patients may experience decreased appetite.

Anorexia nervosa (or nervous asitia, apocleisis), is a disease exhibiting psychotic symptoms, such as a characteristic desire for emaciation and an abnormal eating behavior, as well as somatic symptoms, such as weight loss of 20 % or more of the standard body weight, as well as amenorrhea (in women). It develops frequently in juvenile women and is a serious, and sometimes fatal disease.

Very few compounds are known in the art to be useful for treating eating disorders, such as those described above. Moreover, these therapeutic regimens suffer from numerous problems and a more effective, physiological way to treat eating disorders is highly desirable.

Growth hormone, which is secreted from the pituitary, stimulates growth of all tissues of the body that are capable of growing. In addition, growth hormone is known to have the following basic effects on the metabolic processes of the body: (1) increased rate of protein synthesis in all cells of the body; (2) decreased rate of carbohydrate utilization in cells of the body; and (3) increased mobilization of free fatty acids and use of fatty acids for energy. As is known to those skilled in the art, the known and potential uses of growth hormone are varied and multitudinous. See "Human Growth Hormone," Strobel and Thomas, Pharmacological Reviews, 46, pg. 1-34 (1994). Also, these varied uses of growth hormone are summarized in International Patent Application, Publication Number WO 97/24369.

Various ways are known to release growth hormone (see Recent Progress in Hormone Research, vol. 52, pp. 215-245 (1997); and Front Horm Res. Basel, Karger, vol. 24, pp. 152-175 (1999)). For example, chemicals such as arginine, L-3,4-dihydroxyphenylalanine (L-DOPA), glucagon, vasopressin, and insulin induced hypoglycemia, as well as activities such as sleep and exercise, indirectly cause growth hormone to be released from the pituitary by acting in some fashion on the hypothalamus perhaps either to decrease somatostatin secretion or to increase secretion of growth hormone releasing factor (GRF) or ghrelin (see Nature, vol. 402, pp. 656-660 (9 December 1999)), or all of these.

In cases where increased levels of growth hormone were desired, the problem was generally solved by providing exogenous growth hormone or by administering GRF, IGF-I or a peptidyl compound which stimulated growth hormone production and/or release. In any case, the peptidyl nature of the compound necessitated that it be administered by injection. Initially, the source of growth hormone was the extraction of the pituitary glands of cadavers. This resulted in a very expensive product and carried with it the risk that a disease associated with the source of the pituitary gland could be transmitted to the recipient of the growth hormone. Recombinant growth hormone has become available which, while no longer carrying any risk of disease transmission, is still a very expensive product which must be given by injection. In addition, administration of exogenous growth hormone may result in side-effects, including edema, and does not correlate with the pulsatile release seen in the endogenous release of growth hormone.

Certain compounds have been developed which stimulate the release of endogenous growth hormone. Peptides which are known to stimulate the release of endogenous growth hormone include growth hormone releasing hormone and its analogs, the growth hormone releasing peptides, GHRP-6 and GHRP-1 (described in U.S. Patent No. 4,411,890; International Patent Application, Publication No. WO 89/07110; and International Patent Application, Publication No. WO 89/07111), and GHRP-2 (described in International Patent Application, Publication No. WO 93/04081), as well as hexarelin (J. Endocrinol. Invest., 15 (Suppl. 4): 45 (1992)). Other compounds possessing growth hormone secretagogue activity are disclosed in the following International Patent Applications (listed by Publication Nos.), issued U.S. Patents and published European Patent Applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

In addition, the following growth hormone secretagogues are known in the art: MK-0677, L-162752 and L-163022 (Merck); NN703 and ipamorelin (Novo Nordisk); hexarelin (Pharmacia); GPA-748 (KP102, GHRP-2) (American Home Products); and LY444711 (Eli Lilly). The following agents that stimulate GH release via GHRH/GRF receptor (including GHRH/GRF derivatives, analogs and mimetics) are known in the art: Geref (Ares/Serono); GHRH (1-44) (BioNebraska); Somatorelin (GRF 1-44) (Fujisawa/ICN); and ThGRF (Theratechnologies).

Endocrine Reviews 18(5): 621-645 (1997) provides an overview of peptidomimetic regulation of growth hormone secretion by growth hormone secretagogues. Horm. Res. 1999; 51(suppl 3):16-20 (1999), examines the clinical and experimental effects of growth hormone secretagogues on various organ systems.

International Patent Applications, Publication Nos. WO 97/24369 and WO 98/58947 disclose that certain growth hormone secretagogues are useful for the treatment or prevention of osteoporosis, congestive heart failure, frailty associated with aging, obesity, accelerating bone fracture repair, attenuating protein catabolic response after a major operation, reducing cachexia and protein loss due to chronic illness, accelerating wound healing or accelerating the recovery of burn patients or patients having undergone major surgery, improving muscle strength, mobility, maintenance of skin thickness, metabolic homeostasis or renal homeostasis. Published European patent application 0995748 discloses that certain dipeptide growth hormone secretagogues are useful for the treatment or prevention of musculoskeletal frailty, including osteoporosis.

The administration of a growth hormone secretagogue is also known to enhance the quality of sleep, which is disclosed in International Patent Application, Publication No. WO 97/24369. Commonly assigned U.S. nonprovisional patent application 09/290985, filed 13 April 1999, discloses pharmaceutical compositions comprising certain ₃ adrenergic agonists and growth hormone secretagogues or growth hormone, and their use for treating diabetes, obesity, hyperglycemia, frailty associated with obesity or frailty associated with aging, and for enhancing the quality of sleep in a mammal. International Patent Application, Publication No. WO 98/58949, discloses the treatment of insulin resistance with certain growth hormone secretagogues.

Published European Patent Application 0 916 345 A1discloses that recombinant growth hormone treatment improves eating behavior, normalizes IGF-1 levels and increases body weight in young women with anorexia nervosa.

W. Locke, H.D. Kirgis, C.Y. Bowers and A.A. Abdoh, Life Sci., 56: 1347-1352 (1995) discloses that intracerebroventricular growth-hormone-releasing-peptide-6 stimulated eating without affecting plasma growth hormone responses in rats.

K. Okada, S. Ishii, S. Minami, H. Sugihara, T. Shibasaki and I. Wakabayashi, Endocrinology, 137: 5155-5158 (1996) discloses that intracerebroventricular administration of growth hormone releasing peptide KP-102 increased food intake in free-feeding rats.

A. Torsello et al., European Journal of Pharmacology 360 (1998) 123-129 (1998), discloses that two well-known hexapeptides, GHRP-6 and hexarelin, given s.c., dose dependently stimulated both GH release and feeding behavior in satiated rats. However, in a series of tri-, penta- and hexapeptide analogs of hexarelin, some compounds were active either on GH release or on eating behavior.

Greet Van den Berghe et al., J. Clin. Endocrinol. Metab. 82: 590-599, 1997; and Greet Van den Berghe et al., J. Clin. Endocrinol. Metab. 83: 1827-1834, 1998; disclose findings that open perspectives for GH secretagogues as potential antagonists of the catabolic state in prolonged critical illness.

R.C. Jenkins, R.J.M. Ross (eds): The Endocrine Response to Acute Illness. Front. Horm. Res. Basel, Karger, 1999. Vol. 24, pp. 152-175, concludes that potential therapeutic indications for the use of growth hormone or growth hormone secretagogues are catabolic states and possibly critical illness.

Drug Discovery Today, Vol. 4, No. 11, November 1999; and TEM Vol. 10, No. 1, 1999, disclose potential therapeutic applications of growth hormone secretagogues, including their use in treating growth hormone disorders such as growth hormone deficiency (GHD), age-related conditions, obesity and catabolic conditions, and their use in sleep enhancement.

J. Clin. Endocrinol. Metab. 83: 320-325, 1998, discloses that MK-677, an orally active growth hormone secretagogue, reverses diet-induced catabolism.

### SUMMARY OF THE INVENTION

The present invention provides methods for increasing or stimulating appetite in a patient comprising the administration of certain growth hormone secretagogues. More preferably, the present invention provides methods for stimulating or increasing appetite in a patient which comprises administering to the patient an appetite stimulating or increasing effective amount of a growth hormone secretagogue, which is a compound of the Formula I: 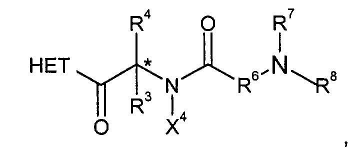 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug, or a tautomer thereof, wherein
HET is a heterocyclic moiety selected from the group consisting of 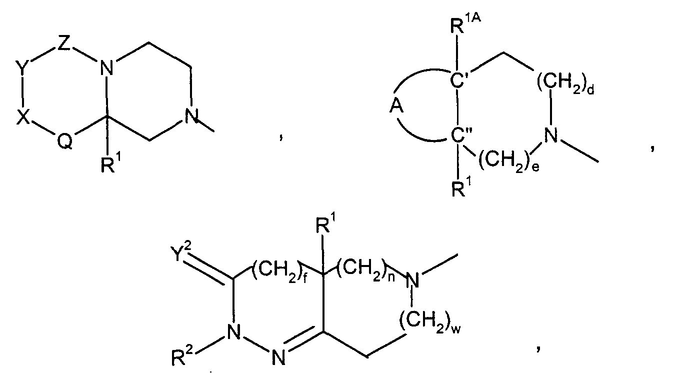 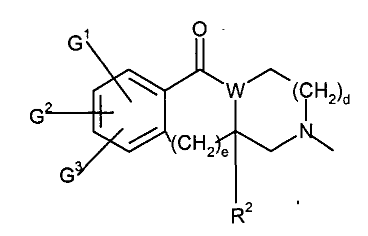 and 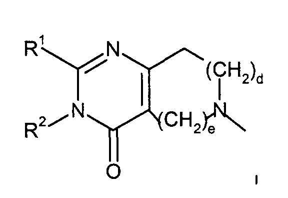
d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
   -NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-,-NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino,-(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
   Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
   q is 0, 1, 2, 3 or 4;
   t is 0, 1, 2 or 3;
   said (CH₂)_{q} group and (CH₂)ₜ group in the definition of R¹ are optionally independently substituted with hydroxy, (C₁-C₄)alkoxy, carboxyl, -CONH₂,
   -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxy, -C(O)OX⁶, -C(O)N(X⁶)(X⁶), -N(X⁶)(X⁶),-S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
   where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
   X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 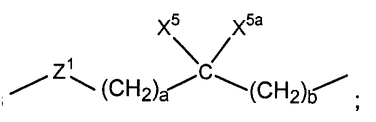 where a and b are each independently 0, 1, 2 or 3;
   X⁵ and X^{5a} are each independently selected from the group consisting of hydrogen, CF₃, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C3-C₇)cycloalkyl, -N(X²)(X²) and -C(O)N(X²)(X²);
   or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
   or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
   or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
   Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the-(CR^{a}R^{b})_{b} group is attached to the terminal nitrogen atom of the compound of Formula I;
   E is -O-, -S-, -CH=CH- or an aromatic moiety selected from 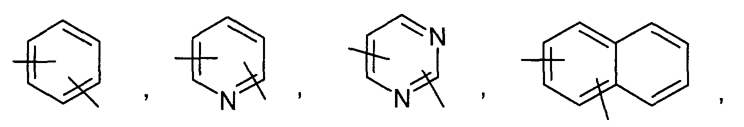 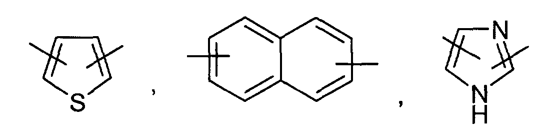 or 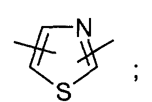 said aromatic moiety in the definition of E optionally substituted with up to three halo, hydroxy, -N(R^{c})(R^{c}), (C₁-C₆)alkyl or (C₁-C₆)alkoxy;
   R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
   R^{c}, for each occurrence, is independently hydrogen or (C₁-C₆)alkyl;
   a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
   where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4-to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6- membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
   A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
      where X¹¹ is hydrogen or optionally substituted (C₁-C₆)alkyl;
         the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
      X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
      or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1 H-tetrazol-5-yl; or when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
   X⁷ is hydrogen or (C₁-C₆)alkyl optionally substituted with hydroxy;
m for each occurrence is independently 0, 1 or 2;
   with the provisos that:
   1) X⁶ and X¹² cannot be hydrogen when attached to C(O) or S(O)₂ in the form C(O)X⁶, C(O)X¹², S(O)₂X⁶ or S(O)₂X¹²; and
   2) when R⁶ is a bond then L is N(X²) and each r in the definition -(CH₂)ᵣ-L-(CH₂)ᵣ- is independently 2 or 3.

More preferably, the present invention provides such methods wherein the compound has the structural formula below, which is designated herein as Formula I-A 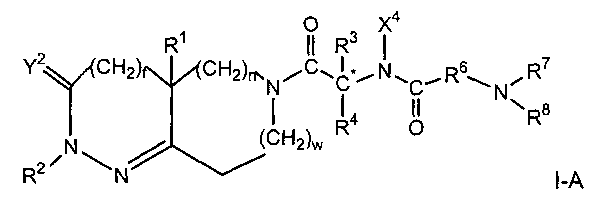 a racemic-diastereomeric mixture or optical isomer of said compound or a pharmaceutically-acceptable salt or a prodrug thereof, or a tautomer thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, (CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
   Y¹ is O, S(O)ₘ, -C(O)NX⁶-, -CH=CH-, -C≡C-, -N(X⁶)C(O)-, -C(O)O-, -OC(O)N(X⁶)- or -OC(O)-;
   q is 0, 1, 2, 3 or 4;
   t is 0, 1, 2 or 3;
   said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ or -(C₁-C₅)alkyl-X¹-(C₁-C₅)alkyl-(C₃-C₇)cycloalkyl;
   where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³;
   X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 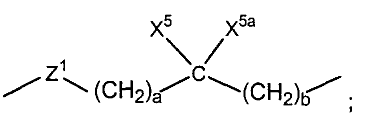
   where a and b are independently 0, 1, 2 or 3;
   X⁵ and X^{5a} are each independently selected from the group consisting of hydrogen, trifluoromethyl, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
   where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8- membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
   A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
   where X¹¹ is hydrogen or optionally substituted (C₁-C₆)alkyl;
      the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
   X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
   or X¹¹ and X¹² are taken together to form -(CH₂)ᵣ-L¹-(CH₂)ᵣ-;
      where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1 H-tetrazol-5-yl; or when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
   X⁷ is hydrogen or (C₁-C₆)alkyl optionally substituted with hydroxyl; and
m for each occurrence is independently 0, 1 or 2;
   with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

In addition, the present invention provides methods for treating an eating disorder in a patient which comprises administering to the patient an eating disorder treating effective amount of certain growth hormone secretagogues. More preferably, the present invention provides methods for treating an eating disorder in a patient which comprises administering to the patient an eating disorder treating effective amount of a growth hormone secretagogue, which is a compound of the Formula I, wherein the variables are as defined above. More preferably, the present invention provides such methods wherein the compound is a compound of Formula I-A, wherein the variables are as defined above.

More preferably, the present invention provides the above methods wherein the compound is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of the compound or the prodrug. Even more preferably, the present invention provides such methods wherein the compound is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate.

Also, more preferably, the present invention provides the above methods wherein the compound is 2-amino-N-(1-(R)-(2,4-difluoro-benzyloxymethyl)-2-oxo-2-(3-oxo-3a-(R)-pyridin-2-ylmethyl-2-(2,2,2-trifluoro-ethyl)-2,3,3a,4,6,7-hexahydropyrazolo-[4,3-c]pyridin-5-yl)-ethyl)-2-methyl-propionamide, a prodrug thereof or a pharmaceutically acceptable salt of the compound or the prodrug. Even more preferably, the present invention provides such methods wherein the compound is the (L)-(+)-tartaric acid salt of 2-amino-N-(1-(R)-(2,4-difluoro-benzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide.

Also, more preferably, the present invention provides the above methods wherein the compound is 2-amino-N-(1-(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, a prodrug thereof or a pharmaceutically acceptable salt of the compound or the prodrug. Even more preferably, the present invention provides such methods wherein the compound is the (L)-(+)-tartaric acid salt of 2-amino-N-(1-(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methylpropionamide.

Also, the present invention provides the above methods, which further comprise administering a recombinant growth hormone or a growth hormone secretagogue selected from the group consisting of GHRP-6, GHRP-1, GHRP-2, hexarelin, growth hormone releasing factor, an analog of growth hormone releasing factor, IGF-I and IGF-II.

Also, the present invention provides the above methods, which further comprise administering an antidepressant, a prodrug thereof or a pharmaceutically acceptable salt of said antidepressant or said prodrug. More particularly, the present invention provides such methods wherein said antidepressant is a norepinephrine reuptake inhibitor (NERI), selective serotonin reuptake inhibitor (SSRI), monoamine oxidase inhibitor (MAO), combined NERI/SSRI, or an atypical antidepressant, a prodrug of said antidepressant or a pharmaceutically acceptable salt of said antidepressant or said prodrug. Even more particularly, the present invention provides such methods wherein said antidepressant is a selective serotonin reuptake inhibitor (SSRI), a prodrug thereof or a pharmaceutically acceptable salt of said SSRI or said prodrug. Even more particularly, the present invention provides such methods wherein said SSRI is citalopram, femoxetine, fluoxetine, fluvoxamine, indalpine, indeloxazine, milnacipran, paroxetine, sertraline, sibutramine or zimeldine, a prodrug of said SSRI or a pharmaceutically acceptable salt of said SSRI or said prodrug. Most particularly, the present invention provides such methods wherein said SSRI is sertraline, a prodrug thereof or a pharmaceutically acceptable salt of sertraline or said prodrug.

In addition, the present invention provides the above methods, which further comprise administering an antiemetic agent, a prodrug thereof or a pharmaceutically acceptable salt of said antiemetic or said prodrug. More particularly, the present invention provides such methods wherein the antiemetic agent is meclizine hydrochloride, prochlorperazine, promethazine, trimethobenzamide hydrochloride or ondansetron hydrochloride.

Finally, the present invention provides the above methods, which further comprise administering an antipsychotic agent, a prodrug thereof or a pharmaceutically acceptable salt of said antipsychotic agent or said prodrug. More particularly, the present invention provides such methods wherein the antipsychotic agent is chlorpromazine, haloperidol, clozapine, loxapine, molindone hydrochloride, thiothixene, olanzapine, ziprasidone, ziprasidone hydrochloride, prochlorperazine, perphenazine, trifluoperazine hydrochloride or risperidone.

### DETAILED DESCRIPTION OF THE INVENTION

The present invention is directed to the use of a compound, which has the ability to stimulate or amplify the release of endogenous growth hormone, for increasing or stimulating appetite. In particular, the present invention provides methods for increasing or stimulating appetite in a patient comprising the administration of certain growth hormone secretagogues.

According to the present invention, certain growth hormone secretagogues are appetite stimulants and thus useful for treating eating disorders. An example of an eating disorder is anorexia nervosa, which most often occurs in young women. It is diagnosed generally based on the following clinical findings: abnormal weight loss by 20% or more of standard body weight, abnormal eating behavior (apocleisis, vomiting, eating in secret, hyperphagia and the like), obsessed recognition with regard to body weight or body shape, onset of age 30 years old or younger, amenorrhea (in women), and absence of organic disease causative of emaciation (such as schizophrenia and depression). It is a serious, and sometimes fatal disease.

As appetite stimulants, growth hormone secretagogues of the present invention are also useful for treating malnutrition. For example, elderly people often suffer from malnutrition due to poor appetite, which is common in the elderly. Other individuals may have decreased appetite due to underlying chronic or acute diseases or conditions or the treatment therefor. For example, patients undergoing cancer chemotherapy, patients who are immunocompromised due to, for example, AIDS, or patients who are immunosuppressed due to, for example, organ transplants, commonly experience decreased appetite. Also, postoperative patients may experience decreased appetite.

Poor appetite can cause low nutrient intake that increases the health risk faced by humans and other animals, especially livestock. For example, *post partum* sows and dairy cows may suffer from reduced feeding behavior. This has a negative effect on lactation and reproductive performance. Shipping and crowding stress can also reduce an animal's nutrient intake, causing deleterious effects that may persist throughout the animal's life.

Humans, livestock and companion animals, which have the following diseases, may also benefit from treatment with appetite stimulants, for example: dental problems, oral tumors, cancer, sepsis, inflammatory bowel disease, chronic pain, cardiac disease, respiratory disease, renal disease, infectious disease, certain antibiotic therapies, post-operative recovery, psychological stress (boarding and moving in animals), geriatric debilitation, pancreatitis (especially in cats), hepatic lipidosis (especially in cats), lymphocytic/plasmocytic stomatitis (especially in cats) and diabetes (especially in cats).

Currently available pharmaceutical therapies have poor efficacy, short duration of effect and can have unattractive side effects. Diazepam is widely used, but effects are transient in dogs and delayed and transient in cats. It cannot be used in patients with liver disease due to severe hepatic necrosis following short-term administration.

Cyproheptadine is more effective in cats than dogs, but has 24-36 hour delay in action, effects are transient, and it is not widely used. Cimetidine and metocloprimide induce gastric emptying and have slight efficacy for some patients. Anecdotal evidence suggests some appetite stimulatory effects of anabolic steroids. These compounds are not widely prescribed for appetite stimulation.

The growth hormone secretagogues of the present invention may benefit the medical conditions described above by stimulating appetite as well as by improving some of the underlying endocrine or metabolic dysfunction.

By the term "growth hormone secretagogue" is meant any exogenously administered compound or agent that directly or indirectly stimulates or increases the endogenous release of growth hormone, growth hormone-releasing hormone or somatostatin in an animal, in particular, a human. This term shall at all times be understood to include all active forms of such secretagogues, including, for example, the free form thereof, e.g., the free acid or base form, and also, all prodrugs, polymorphs, hydrates, solvates, stereoisomers, e.g., diastereomers and enantiomers, and the like, and all pharmaceutically acceptable salts as described above, unless specifically stated otherwise. It will also be appreciated that suitable active metabolites of secretagogues within the scope of the present invention, in any suitable form, are also included herein.

The growth hormone secretagogue may be peptidyl or non-peptidyl in nature, however, the use of an orally active growth hormone secretagogue is preferred. In addition, it is preferred that the growth hormone secretagogue induce or amplify a pulsatile release of endogenous growth hormone.

The expression "prodrug" refers to compounds that are drug precursors which, following administration, release the drug *in vivo* via some chemical or physiological process (e.g., a prodrug on being brought to the physiological pH is converted to the desired drug form). For example, a prodrug of the compound of Formula I may be used in the present invention. Exemplary prodrugs are disclosed in the art, particularly in the references cited herein and incorporated herein by reference.

The growth hormone secretagogue of the present invention may be used alone or in combination with one or more other growth hormone secretagogues or with one or more other agents which are known to be beneficial for increasing appetite. The growth hormone secretagogue and the other agent may be coadministered, either in concomitant therapy or in a fixed combination.

Representative growth hormone secretagogues are disclosed in the following International Patent Applications (listed by Publication Nos.), issued U.S. patents and published European patent applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A representative first group of growth hormone secretagogues is set forth in International Patent Application, Publication No. WO 97/24369, as compounds having the structural formula below, which is designated herein as Formula II: 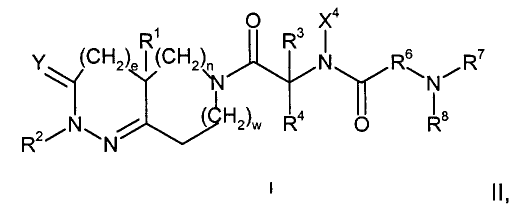 wherein the various substituents are as defined in WO 97/24369. Said compounds are prepared as disclosed therein.

2-Amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide, having the following structure: 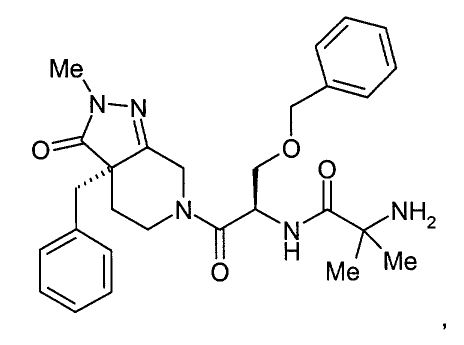 and 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin5-yl)-ethyl)-2-methyl-propionamide, having the following structure: 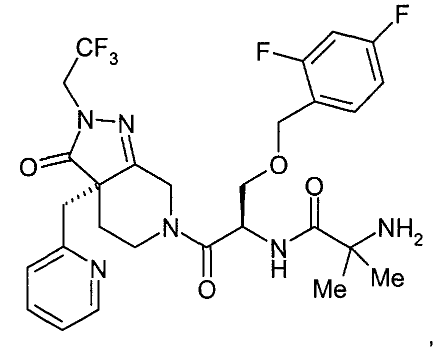 and the pharmaceutically acceptable salts thereof, are within the scope of the disclosure of International Patent Application, Publication Number WO 97/24369.

A representative second group of growth hormone secretagogues is set forth in International Patent Application, Publication No. WO 98/58947, as compounds having the structural formula below, which is designated herein as Formula III: 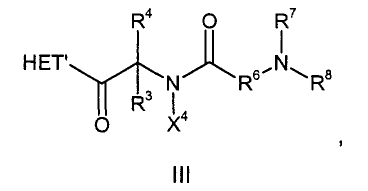 wherein the various substituents are as defined in WO 98/58947. Said compounds are prepared as disclosed therein.

A most preferred compound within this second group which may be employed in the present invention is identified as having the following name and structure: 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, 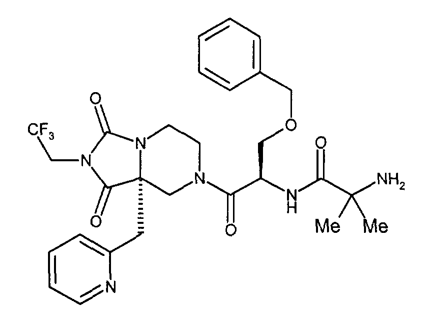 This compound and the pharmaceutically acceptable salts thereof are within the scope of the disclosure of International Patent Application, Publication No. WO 98/58947, and may be prepared as described in Examples Five and Six therein.

A representative third group of growth hormone secretagogues is set forth in Published European patent application 0995748, which discloses certain dipeptide growth hormone secretagogues of the structural formula above, which is designated herein as Formula III, and their use for the treatment or prevention of musculoskeletal fraility including osteoporosis.

A representative fourth group of growth hormone secretagogues is set forth in U.S. Patent No. 5,206,235 as having the following structure: 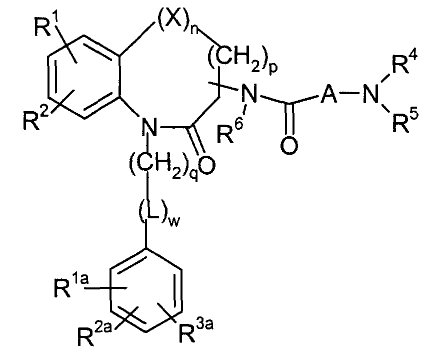 wherein the various substituents are as defined in U.S. Patent No. 5,206,235. Said compounds are prepared as disclosed therein.

Preferred compounds within this fourth group are identified as having the following structures: 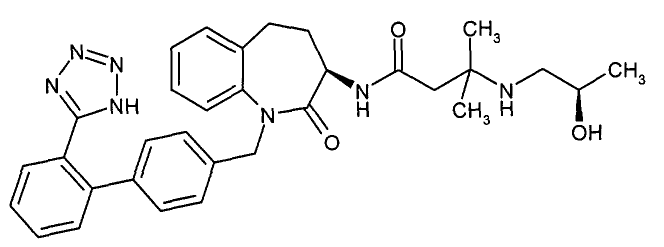 and

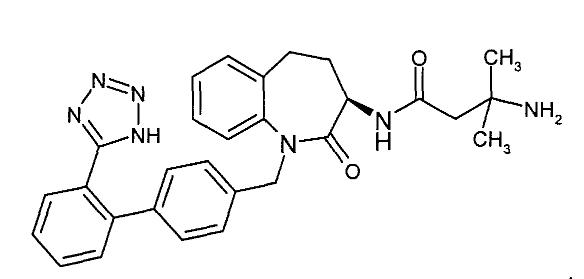

A representative fifth group of growth hormone secretagogues is set forth in U.S. Patent No. 5,283,241 as having the following structural formula: 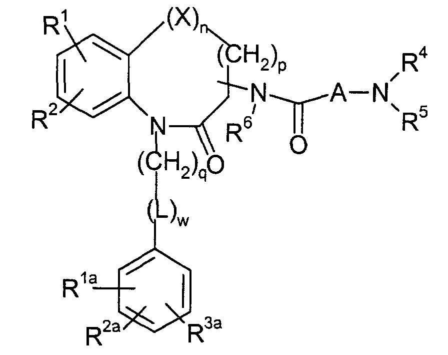 wherein the various substituents are as defined in U.S. Patent 5,283,241. Said compounds are prepared as disclosed therein.

A representative sixth group of growth hormone secretagogues is disclosed in International Patent Application, Publication No. WO 97/41879, as compounds having the following structural formulas: 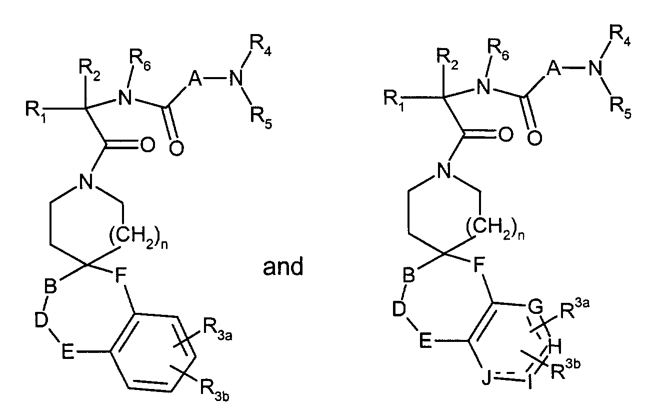 wherein the various substituents are as defined in WO97/41879. Said compounds are prepared as disclosed therein.

Preferred compounds within this sixth group are identified as having the following structure: 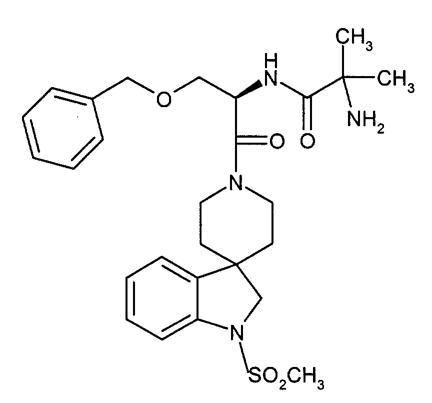 and pharmaceutically acceptable salts thereof, in particular, the methanesulfonate salt.

A representative seventh group of growth hormone secretagogues is disclosed in U.S. Patent No. 5,492,916, as being compounds of the following structural formula: 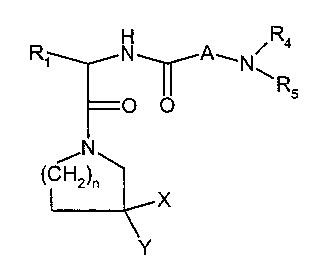 wherein the various substituents are as defined in U.S. Patent No. 5,492,916. Said compounds are prepared as disclosed therein.

All of the compounds identified above may be prepared by procedures disclosed in the cited publications. Full descriptions of the preparation of the growth hormone secretagogues which may be employed in the present invention may be found in the art, particularly in the references cited herein, which are incorporated by reference herein.

The compounds of Formula I used in the methods of the present invention all have at least one asymmetric center as noted, e.g., by the asterisk in the structural Formula I-B below. Additional asymmetric centers may be present in the compounds of Formula I depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, be included within the scope of the methods of the present invention. In the case of the asymmetric center represented by the asterisk, it has been found that the absolute stereochemistry of the more active and thus more preferred isomer is shown in Formula I-B below: 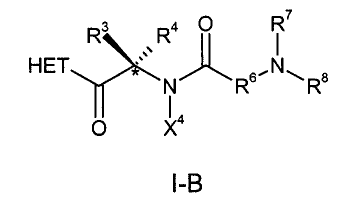 With the R⁴ substituent as hydrogen, the spatial configuration of the asymmetric center corresponds to that in a D-amino acid. In most cases this is also designated an R-configuration although this will vary according to the values of R³ and R⁴ used in making R- or S-stereochemical assignments.

Certain compounds within the scope of the present invention may have the potential to exist in different tautomeric forms. All tautomers of a compound of the present invention are within the scope of the present invention. Also, for example, all keto-enol or imine-enamine forms of the compounds are included in the present invention. Those skilled in the art will recognize that the compound names contained herein may be based on a particular tautomer of a compound. While the name for only a particular tautomer may be used, it is intended that all tautomers are encompassed by the name of the particular tautomer and all tautomers are considered part of the present invention.

A compound within the scope of the present invention may exist in unsolvated as well as solvated forms with pharmaceutically acceptable solvents such as water, ethanol, and the like. A solvate wherein the solvent is water forms a hydrate or hydrated ions. The present invention contemplates and encompasses both the solvated and unsolvated forms of the compounds within its scope.

Also included within the scope of the present invention are isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁵Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in compound and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labelled compounds of the present invention and prodrugs thereof can generally be prepared by carrying out the procedures disclosed in the references cited herein as well as others known in the art, by substituting a readily available isotopically labelled reagent for a non-isotopically labelled reagent.

Full descriptions of the preparation of the growth hormone secretagogues employed in the present invention may be found, for example, in the following International Patent Applications (listed by Publication Nos.), issued U.S. patents and published European patent applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A growth hormone secretagogue is a compound that, when administered to a patient, increases the production and/or secretion of growth hormone when compared with baseline plasma concentrations of growth hormone in a normal healthy individual. Thus, to identify a growth hormone secretagogue, one need simply measure the baseline plasma concentrations of growth hormone over a time period, typically one day, and compare the plasma concentrations of growth hormone after administration of a growth hormone secretagogue with the baseline concentration over the time period. Various examples of growth hormone secretagogues are disclosed herein. It is contemplated that a growth hormone secretagogue of the present invention can be used in the present administration methods.

The identification of a compound as a "growth hormone secretagogue" which is able to directly or indirectly stimulate or increase the endogenous release of growth hormone in an animal may be readily determined without undue experimentation by methodology well known in the art, such as the assay described by Smith et al., Science, 260, 1640-1643 (1993) (see text of Figure 2 therein). In a typical experiment, pituitary glands are aseptically removed from 150-200 g Wistar male rats and cultures of pituitary cells are prepared according to Cheng et al., Endocrinol., 124, 2791-2798 (1989). The cells are treated with the subject compound and assayed for growth hormone secreting activity, as described by Cheng et al. (ibid.). In particular, the intrinsic growth hormone secretagogue activity of a compound which may be used in the present invention may be determined by this assay.

The term "patient" means an animal, such as a human, a companion animal such as a dog, cat and horse, and livestock such as cattle, swine and sheep. Particularly preferred patients are mammals, including both males and females, with humans being even more preferred.

The term "pharmaceutically acceptable" means that a substance or mixture of substances must be compatible with the other ingredients of a formulation, and not deleterious to the patient.

The terms "treating", "treat" or "treatment" include preventive (e.g., prophylactic) and palliative treatment.

The term "therapeutically effective amount" means an amount of a growth hormone secretagogue that ameliorates, attenuates, or eliminates a particular disease or condition associated with growth hormone secretion and/or production, or prevents or delays the onset of a disease or condition associated with growth hormone secretion and/or production.

The phrases "a compound of the present invention or a compound of Formula I" and the like, shall at all times be understood to include all active forms of such compounds, including, for example, the free form thereof, e.g., the free acid or base form, and also, all prodrugs, polymorphs, hydrates, solvates, stereoisomers, e.g., diastereomers and enantiomers, and the like, and all pharmaceutically acceptable salts as described above, unless specifically stated otherwise. It will also be appreciated that suitable active metabolites of compounds within the scope of the present invention, in any suitable form, are also included herein.

This particular application of growth hormone secretagogues provides benefits relative to the administration of exogenous growth hormone. In particular, the growth hormone secretagogue enhances the normal pulsatile release of endogenous growth hormone and thus is more likely to reproduce the natural pattern of endogenous growth hormone release (see J. Clin. Endocrinol. Metab. 81: 4249-4257, 1996). Growth hormone secretagogues which are orally active also have the benefit of being able to be administered orally, rather than just intravenously, intraperitoneally or subcutaneously.

In view of their use according to the present invention, the growth hormone secretagogues of the present invention may be formulated into various pharmaceutical forms for administration purposes. A growth hormone secretagogue may be administered, alone or in combination, by oral, parenteral (e.g., intramuscular, intraperitoneal, intravenous or subcutaneous injection, or implant), nasal, vaginal, rectal, sublingual, or topical routes of administration and can be formulated with pharmaceutically acceptable carriers to provide dosage forms appropriate for each route of administration.

Solid dosage forms for oral administration include capsules, tablets, pills, powders and granules and for companion animals the solid dosage forms include an admixture with food and chewable forms. In such solid dosage forms, the compounds and combinations of this invention can be admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings. In the case of chewable forms, the dosage form may comprise flavoring agents and perfuming agents.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the compound of the present invention, excipients such as cocoa butter or a suppository wax. Compositions for nasal or sublingual administration are also prepared with standard excipients well known in the art.

The dosage of the compound of the present invention in the compositions, methods and combinations of the present invention may be varied; however, it is necessary that the amount of the compound be such that a suitable dosage form is obtained. The selected dosage depends upon the desired therapeutic effect, on the route of administration, and on the duration of the treatment. Generally, dosage levels of between 0.0001 to 100 mg/kg of body weight daily are administered to humans and other animals, e.g., mammals, to obtain effective release of growth hormone. A preferred dosage range in humans is 0.01 to 5.0 mg/kg of body weight daily which can be administered as a single dose or divided into multiple doses.

A preferred dosage range in animals other than humans is 0.01 to 10.0 mg/kg of body weight daily which can be administered as a single dose or divided into multiple doses. A more preferred dosage range in animals other than humans is 0.1 to 5 mg/kg of body weight daily which can be administered as a single dose or divided into multiple doses.

Where the tartrate salt or other pharmaceutically acceptable salt of the compound(s) of the present invention is used, the skilled person will be able to calculate effective dosage amounts by calculating the molecular weight of the salt form and performing simple stoichiometric ratios.

The present invention includes within its scope the use of a growth hormone secretagogue according to the present invention, alone or in combination with a growth promoting agent or another growth hormone secretagogue, such as those referenced herein, including the growth hormone releasing peptides GHRP-6 and GHRP-1 (described in U.S. Patent No. 4,411,890 and International Patent Applications, Publication Nos. WO 89/07110, WO 89/07111), GHRP-2 (described in WO 93/04081) and B-HT920, as well as hexarelin and growth hormone releasing hormone (GHRH, also designated GRF) and its analogs, growth hormone, natural or recombinant, and its analogs and somatomedins including IGF-I and IGF-II, or in combination with other therapeutic agents, such as -adrenergic agonists such as clonidine or serotonin 5-HT1 D agonists such as sumatriptan, or agents which inhibit somatostatin or its release such as physostigmine and pyridostigmine. Preferably, the growth hormone secretagogue may be used in combination with growth hormone releasing factor, an analog of growth hormone releasing factor, IGF-I or IGF-II.

Methods to obtain the growth hormone releasing peptides GHRP-6 and GHRP-1 are described in U.S. Patent Nos. 4,411, 890 and PCT Patent Publications WO 89/07110, WO 89/07111, methods to obtain the growth hormone releasing peptide GHRP-2 are described in PCT Patent Publication WO 93/04081, and methods to obtain hexarelin are described in J. Endocrin. Invest., 15 (Suppl. 4), 45 (1992), all of which are incorporated herein by reference.

Also, the present invention includes within its scope the use of a growth hormone secretagogue according to the present invention, alone or in combination with an antidepressant, a prodrug thereof or a pharmaceutically acceptable salt of said antidepressant or said prodrug. Any antidepressant may be used in the methods of the present invention. The term antidepressant means an agent used to treat affective or mood disorders and related conditions. Affective mood disorders are characterized by changes in mood as the primary clinical manifestation. Either extreme of mood may be associated with psychosis, manifested as disordered or delusional thinking and perceptions which are often incongruent with the predominant mood. Affective disorders include major depression and mania, including bipolar manic-depressive illness. Preferred classes of antidepressants include norepinephrine reuptake inhibitors (NERIs), including secondary and tertiary amine tricyclics; selective serotonin reuptake inhibitors (SSRIs); combined NERI/SSRIs; monoamine oxidase (MAO) inhibitors; and atypical antidepressants.

Any norepinephrine reuptake inhibitor (NERI) may be used in the methods of the present invention. The term norepinephrine reuptake inhibitor means agents which potentiate the actions of biogenic amines by blocking their major means of physiological inactivation, which involves transport or reuptake into nerve terminals, and specifically, agents which block the reuptake of norepinephrine into said nerve terminals.

Preferred tertiary amine tricyclic norepinephrine reuptake inhibitors which may be used in accordance with the present invention include, but are not limited to, amitriptyline, which may be prepared as described in United States Patent No. 3,205,264; chlomipramine, which may be prepared as described in United States Patent No. 3,467,650; doxepin, which may be prepared as described in United States Patent No. 3,420,851; imipramine, which may be prepared as described in United States Patent No. 2,554,736; and trimipramine, which may be prepared as described in Jacob and Messer, *Compt. Rend.* 252, 2117 (1961).

Preferred secondary amine tricyclic norepinephrine reuptake inhibitors which may be used in accordance with the present invention include, but are not limited to, amoxapine, which may be prepared as described in United States Patent No. 3,663,696; desipramine, which may be prepared as described in United States Patent No. 3,454,554; maprotiline, which may be prepared as described in United States Patent No. 3,999,201; nortriptyline, which may be prepared as described in United States Patent No. 3,442,949; and protriptyline, which may be prepared as described in United States Patent No. 3,244,748.

Any selective serotonin reuptake inhibitor (SSRI) may be used in the methods of the present invention. The term selective serotonin reuptake inhibitor refers to a compound which inhibits the reuptake of serotonin by afferent neurons. Such inhibition is readily determined by those skilled in the art according to standard assays such as those disclosed in U.S. 4,536,518 and other U.S. patents recited in the next paragraph.

Preferred selective serotonin reuptake inhibitors (SSRIs) which may be used in accordance with the present invention include, but are not limited to: citalopram, which may be prepared as described in United States Patent No. 4,136,193; femoxetine, which may be prepared as described in United States Patent No. 3,912,743; fluoxetine, which may be prepared as described in United States Patent No. 4,314,081; fluvoxamine, which may be prepared as described in United States Patent No. 4,085,225; indalpine, which may be prepared as described in United States Patent No. 4,064,255; indeloxazine, which may be prepared as described in United States Patent No. 4,109,088; milnacipran, which may be prepared as described in United States Patent No. 4,478,836; paroxetine, which may be prepared as described in United States Patent No. 3,912,743 or United States Patent No. 4,007,196; sertraline and the hydrochloride salt of sertraline, which may be prepared as described in United States Patent No. 4,536,518; sibutramine, which may be prepared as described in United States Patent No. 4,929,629; and zimeldine, which may be prepared as described in United States Patent No. 3,928,369. Fluoxetine is also known as Prozac® . Sertraline hydrochloride is also known as Zoloft® . Sibutramine is also known as Meridia® .

Any combined NERI/SSRI may be used in the methods of the present invention. The term combined NERI/SSRI refers to a compound which blocks the reuptake of both serotonin and norepinephrine by afferent neurons. A preferred combined NERI/SSRI which may be used in accordance with the present invention is venlafaxine, which may be prepared as described in United States Patent No. 4,535,186.

Any monoamine oxidase (MAO) inhibitor may be used in the methods of the present invention. The term monoamine oxidase inhibitor refers to a compound which inhibits monoamine oxidase, for example, by blocking the metabolic deamination of a variety of monoamines by mitochondrial monoamine oxidase. Preferred monoamine oxidase inhibitors, which may be used in accordance with the present invention, include, but are not limited to, phenelzine, which may be prepared as described in United States Patent No. 3,000,903; tranylcypromine, which may be prepared as described in United States Patent No. 2,997,422; and selegiline, which may be prepared as described in United States Patent No. 4,564,706.

Any atypical antidepressant may be used in the methods of the present invention. The term atypical antidepressant refers to any antidepressant not within any of the aforesaid classes of antidepressants. Preferred atypical antidepressants which may be used in accordance with the present invention include, but are not limited to, bupropion, which may be prepared as described in United States Patent No. 3,885,046; nefazodone, which may be prepared as described in United States Patent No. 4,338,317; and trazodone, which may be prepared as described in United States Patent No. 3,381,009.

Also, the present invention includes within its scope the use of a growth hormone secretagogue according to the present invention, alone or in combination with an antiemetic agent, a prodrug thereof or a pharmaceutically acceptable salt of said antiemetic or said prodrug. Many antiemetic agents are used, including D2 antagonists (substituted benzamides, phenothiazines, butyrophenones and benzimidazole derivatives), 5HT₃ antagonists, corticosteroids, cannabinoids, antihistamines, muscarinic antagonists and benzodiazepines. Most of these agents are available for oral and parenteral administration and as suppositories. In addition to the availability of specific receptor antagonists, the use of combinations of agents has represented a major improvement in the ability to reduce this side effect of many chemotherapeutic protocols. Thus, a primary antiemetic therapy may be supplemented with a corticosteroid, providing more efficacious antiemetic therapy and reduced toxicity from the primary agent. Further information on antiemetic agents is provided in Goodman & Gilman's The Pharmacological Basis of Therapeutics, Ninth Edition, McGraw-Hill, New York (1996), pages 928-932, which is incorporated by reference herein. Examples of antiemetic agents include meclizine hydrochloride (Antivert®), prochlorperazine (Compazine®), promethazine (Phenergan®), trimethobenzamide hydrochloride (Tigan®), and ondansetron hydrochloride (Zofran®) (Physician's Desk Reference, 53^{rd} Ed., Medical Economics Co., Inc., Montvale, N.J. (1999)).

Also, the present invention includes within its scope the use of a growth hormone secretagogue according to the present invention, alone or in combination with an antipsychotic agent, a prodrug thereof or a pharmaceutically acceptable salt of said antipsychotic or said prodrug. Antipsychotic agents are used primarily in the management of patients with psychotic or other serious psychiatric illnesses marked by agitation and impaired reasoning. These drugs have other properties that possibly are useful clinically, including antiemetic and antihistaminic effects and the ability to potentiate analgesics, sedatives, and general anesthetics. Effective antipsychotic compounds include the phenothiazines, structurally similar thioxanthenes, and heterocyclic dibenzazepines; the butyrophenones (phenylbutylpiperidines) and diphenylbutylpiperidines; and the indolones and other heterocyclic compounds. Chlorpromazine (Thorazine®) is the oldest representative of the phenothiazine-thioxanthene group of antipsychotic agents, and haloperidol (Haldol®) is the original butyrophenone and representative of several related classes of aromatic butylpiperidine derivatives. Further information on antipsychotic agents is provided in Goodman & Gilman's The Pharmacological Basis of Therapeutics, Ninth Edition, McGraw-Hill, New York (1996), pages 399-430, which is incorporated by reference herein. Examples of other antipsychotic agents include clozapine (Clozaril®), loxapine (Loxitane®), molindone hydrochloride (Moban®), thiothixene (Navane®), olanzapine (Zyprexa®), ziprasidone and its pharmaceutically acceptable salts, such as its hydrochloride, monohydrate (Geodon®), prochlorperazine (Compazine®), perphenazine (Trilafon®), trifluoperazine hydrochloride (Stalazine®) and risperidone (Risperdal®) (Physician's Desk Reference, 53^{rd} Ed., Medical Economics Co., Inc., Montvale, N.J. (1999) and supplements thereto.)

The disclosures of each of the patents and published patent applications cited within this description are incorporated herein by reference.

In addition, the present invention includes within its scope the use of a pharmaceutical composition according to the present invention comprising at least one growth hormone secretagogue of the present invention in association with a pharmaceutical carrier, vehicle or diluent. The present invention also includes within its scope the use of a compound of Formula I for the preparation of a medicament for the uses disclosed herein.

Combinations of these therapeutic agents, some of which have been mentioned herein, with a growth hormone secretagogue of the present invention may bring additional complementary properties to enhance the desirable properties of these various therapeutic agents.

In these combinations, the growth hormone secretagogue and the other therapeutic agent(s) may be independently present in the dose ranges from 0.01 to 1 times the dose levels which are effective when these compounds and secretagogues are used singly.

Typically, the individual daily dosages for these combinations may range from about one-fifth of the minimally recommended clinical dosages to the maximum recommended levels for the entities when they are given singly. These dose ranges may be adjusted on a unit basis as necessary to permit divided daily dosage and, as noted above, the dose will vary depending on the nature and severity of the disease, weight of patient, special diets and other factors.

These combinations may be formulated into pharmaceutical compositions as known in the art and as discussed herein. Since the present invention has an aspect that relates to treatment with a combination of active ingredients which may be administered separately, the invention also relates to combining separate pharmaceutical compositions in kit form. The kit comprises two separate pharmaceutical compositions: a growth hormone secretagogue of the present invention, a prodrug thereof or a pharmaceutically acceptable salt of said growth hormone secretagogue or said prodrug; and a second therapeutic agent as described herein. The kit comprises a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of a second therapeutic agent as described herein can consist of one tablet or capsule while a daily dose of the growth hormone secretagogue, prodrug thereof or pharmaceutically acceptable salt of said growth hormone secretagogue or said prodrug can consist of several tablets or capsules or vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The utility of the compounds described herein in the methods of the present invention are demonstrated by their activity in one or more of the assays described below:

### Assay for Stimulation of Growth Hormone Release from Rat Pituicytes

Compounds having the ability to stimulate GH secretion from cultured rat pituitary cells are identified using the following protocol. This test is also useful for comparison to standards to determine dosage levels.

Cells are isolated from pituitaries of 6-week old male Wistar rats. Following decapitation, the anterior pituitary lobes are removed into cold, sterile Hank's balanced salt solution without calcium or magnesium (HBSS). Tissues are finely minced, then subjected to two cycles of mechanically assisted enzymatic dispersion using 10 U/mL bacterial protease (EC 3.4.24.4, Sigma P-6141, St. Louis, Missouri) in HBSS. The tissue-enzyme mixture is stirred in a spinner flask at 30 rpm in a 5% CO₂ atmosphere at 37 °C for 30 min., with manual trituration after 15 min. and 30 min. using a 10-mL pipet. This mixture is centrifuged at 200 x g for 5 min. Horse serum (35% final concentration) is added to the supernatant to neutralize excess protease. The pellet is resuspended in fresh protease (10 U/mL), stirred for about 30 min. more under the previous conditions, and manually triturated, ultimately through a 23-gauge needle. Again, horse serum (35% final concentration) is added, then the cells from both digests are combined, pelleted (200 x g for about 15 min.), resuspended in culture medium (Dulbecco's Modified Eagle Medium (D-MEM) supplemented with 4.5 g/L glucose, 10% horse serum, 2.5% fetal bovine serum, 1% non-essential amino acids, 100 U/mL nystatin and 50 mg/mL gentamycin sulfate, Gibco, Grand Island, New York) and counted. Cells are plated at 6.0-6.5x10⁴ cells per cm² in 48-well Costar™ (Cambridge, Massachusetts) dishes and cultured for 3-4 days in culture medium.

Just prior to carrying out a GH secretion assay, culture wells are rinsed twice with release medium, then equilibrated for 30 minutes in release medium (D-MEM buffered with 25 mM Hepes, pH 7.4 and containing 0.5% bovine serum albumin at 37 °C). Test compounds are dissolved in DMSO, then diluted into prewarmed release medium. Assays are typically run in quadruplicate. The assay is initiated by adding 0.5 mL of release medium (with vehicle or test compound) to each culture well. Incubation is carried out at 37 °C for 15 minutes, then terminated by removal of the release medium, which is centrifuged at 2000 x g for 15 minutes to remove cellular material. Rat growth hormone concentrations in the supernatants are determined by a standard radioimmunoassay protocol described below.

### Assay for Exogenously-Stimulated Growth Hormone Release in the Rat after Intravenous Administration of Test Compounds

Twenty-one day old female Sprague-Dawley rats (Charles River Laboratory, Wilmington, MA) are allowed to acclimate to local vivarium conditions (24 °C, 12 hr light, 12 hr dark cycle) for approximately 1 week before testing of a compound of this invention. All rats are allowed access to water and a pelleted commercial diet (Agway Country Food, Syracuse NY) *ad libitum.*

On the day of the experiment, test compounds are dissolved in vehicle containing 1% ethanol, 1 mM acetic acid and 0.1% bovine serum albumin in saline. Each test is conducted in three rats. Rats are weighed and anesthetized via intraperitoneal injection of sodium pentobarbital (Nembutol®, 50 mg/kg body weight). Fourteen minutes after anesthetic administration, a blood sample is taken by nicking the tip of the tail and allowing the blood to drip into a microcentrifuge tube (baseline blood sample, approximately 100 µl). Fifteen minutes after anesthetic administration, a test compound is delivered by intravenous injection into the tail vein, with a total injection volume of 1 mL/kg body weight. Additional blood samples are taken from the tail at 5, 10 and 15 minutes after administration of a compound of this invention. Blood samples are kept on ice until serum separation by centrifugation (1430 x g for 10 minutes at 10°C). Serum is stored at -80 °C until serum growth hormone determination by radioimmunoassay as described below.

### Measurement of Rat Growth Hormone

Rat growth hormone concentrations are determined by double antibody radioimmunoassay using a rat growth hormone reference preparation (NIDDK-rGH-RP-2) and rat growth hormone antiserum raised in monkey (NIDDK-anti-rGH-S-5) obtained from Dr. A. Parlow (Harbor-UCLA Medical Center, Torrance, CA). Additional rat growth hormone (1.5U/mg, #G2414, Scripps Labs, San Diego, CA) is iodinated to a specific activity of approximately 30 µCi/µg by the chloramine T method for use as tracer. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg rat growth hormone per tube.

### Assessment of Growth Hormone Release in the Dog after Oral Administration

On the day of dosing, the test compound is weighed out for the appropriate dose and dissolved in water. Doses are delivered at a volume of 0.5-3 mL/kg by oral gavage to 2-4 dogs for each dosing regimen. Blood samples (5 mL) are collected from the jugular vein by direct venipuncture pre-dose and at 0.17, 0.33, 0.5, 0.75, 1, 2, 4, 6, 8 and 24 hours post dose using 5 mL vacutainers containing lithium heparin. The prepared plasma is stored at -20 °C until analysis.

### Measurement of Canine Growth Hormone

Canine growth hormone concentrations are determined by a standard radioimmunoassay protocol using canine growth hormone (antigen for iodination and reference preparation AFP-1983B) and canine growth hormone antiserum raised in monkey (AFP-21452578) obtained from Dr. A. Parlow (Harbor-UCLA Medical Center, Torrence, CA). Tracer is produced by chloramine T-iodination of canine growth hormone to a specific activity of 20-40 µCi/µg. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg canine GH/tube.

### Phase I, Double-Blind, Placebo-Controlled Study of the Clinical Pharmacology of a Test Compound Following Single Oral Escalating Doses in Healthy Young Male Volunteers

This study was a randomized, placebo-controlled, single escalating oral dose study of the clinical pharmacology of the test compound, the (L)-(+)-tartaric acid salt of 2-amino-N-(1-(R)-(2,4-difluoro-benzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide.

At each dose level specified below, six (6) healthy, young male subjects were randomly assigned, on a 2:1 basis (four (4) active: two (2) placebo), to receive either the test compound or placebo, according to a computer generated randomization schedule. The test compound or placebo was administered orally in a solution of water to the subjects in a fasted state. The doses evaluated were 0.3, 1, 3, 10, 30 and 100 mg of the test compound or placebo. Each participant was studied at only one dose level.

On the case report forms, two of the four subjects, who had received a 100 mg dose of the test compound, self-reported feeling hungry.

While the foregoing description discloses the present invention, with examples provided for the purpose of illustration, it will be understood that the practice of the present invention encompasses all of the usual variations, adaptations or modifications as come within the scope of the following claims and their equivalents.

## Claims

1. Use of a compound of the Formula I: 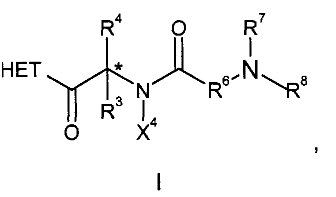 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug, or a tautomer thereof,
wherein:
HET is a heterocyclic moiety selected from the group consisting of 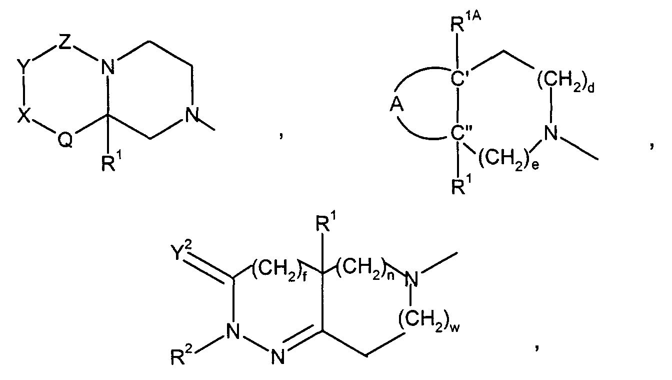 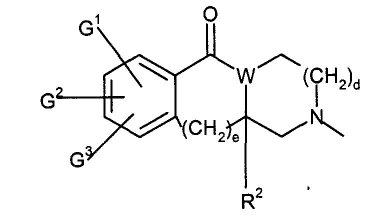 and 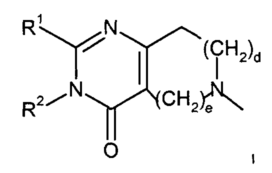
d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
-NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰) -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino,-(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹,
-(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxy, -C(O)OX⁶, -C(O)N(X⁶)(X⁶), -N(X⁶)(X⁶),-S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 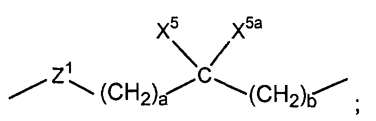
where a and b are each independently 0, 1, 2 or 3;
X⁵ and X^{5a} are each independently selected from the group consisting of hydrogen, CF₃, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C3-C₇)cycloalkyl, -N(X²)(X²) and -C(O)N(X²)(X²);
or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the-(CR^{a}R^{b})_{b} group is attached to the terminal nitrogen atom of the compound of formula I;
E is -O-, -S-, -CH=CH- or an aromatic moiety selected from 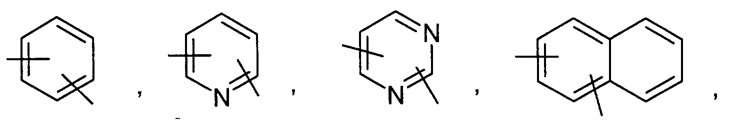 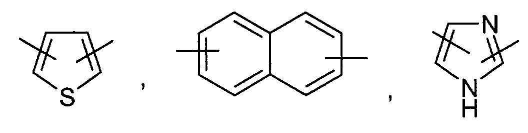 or 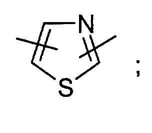
said aromatic moiety in the definition of E optionally substituted with up to three halo, hydroxy, -N(R^{c})(R^{c}), (C₁-C₆)alkyl or (C₁-C₆)alkoxy;
R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
R^{c}, for each occurrence, is independently hydrogen or (C₁-C₆)alkyl;
a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶,
-C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono-or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3;
for the preparation of a medicament for stimulating or increasing appetite in a patient.

2. The use according to claim 1 wherein the compound is of Formula I-A 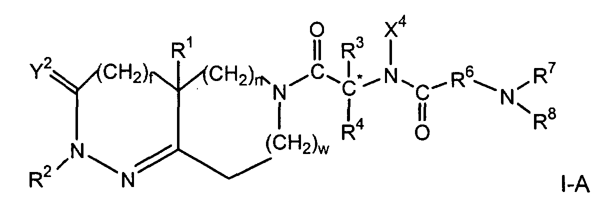 a racemic-diastereomeric mixture or an optical isomer of said compound or a pharmaceutically-acceptable salt or a prodrug thereof, or a tautomer thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹,
-(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
Y¹ is O, S(O)ₘ, -C(O)NX⁶-, -CH=CH-, -C≡C-, -N(X⁶)C(O)-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl,
-CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxyl, -C(O)OX⁶, -C(O)N(X⁶)(X⁶),
-N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl,
-(C₁-C₅)alkyl-X¹-(C₁-C₅)alkyl,-(C₁-C₅)alkyl-X¹-(C₀-C₅)alkyl-A¹ or -(C₁-C₅)alkyl-X¹-(C₁-C₅)alkyl-(C₃-C₇)cycloalkyl;
where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 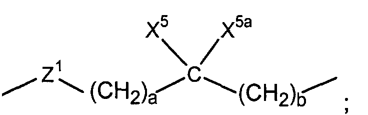
where a and b are independently 0, 1, 2 or 3;
X⁵ and X^{5a} are each independently selected from the group consisting of
hydrogen, trifluoromethyl, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX²,
(C₃-C₇)cycloalkyl, -N(X²)(X²) and -C(O)N(X²)(X²);
R⁷ and R⁸ are independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl,
-S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶,
-C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH3, OCH₃, OCF₃ and CF₃;
or X¹¹ and X¹² are taken together to form -(CH₂)ᵣ-L¹-(CH₂)ᵣ-;
where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
X⁷ is hydrogen or (C₁-C₆)alkyl optionally substituted with hydroxyl; and
m for each occurrence is independently 0, 1 or 2;
with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

3. The use according to claim 1 or 2 wherein the compound, the prodrug thereof or the pharmaceutically acceptable salt of said compound or said prodrug, is selected from the group consisting of:
2-amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide;
2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxo-ethyl]-isobutyramide, L-tartrate;
2-amino-N-(1-(R)-(2,4-difluoro-benzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide; and
the (L)-(+)-tartaric acid salt of 2-amino-N-(1-(R)-(2,4-difluorobenzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide.

4. The use according to claim 1 wherein the compound, the prodrug thereof or the pharmaceutically acceptable salt of said compound or said prodrug, is selected from the group consisting of:
2-amino-N-{1(R)-benzyloxymethyl-2-[1,3-dioxo-8a(S)-pyridin-2-ylmethyl-2-(2,2,2-trifluoro-ethyl)-hexahydro-imidazo[1,5-a]pyrazin-7-yl]-2-oxo-ethyl}-2-methylpropionamide; and
the (L)-(+)-tartaric acid salt of 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide.

5. The use according to any one of claims 1-4 wherein the patient is a human.

6. The use according to claim 5 wherein the human is elderly, acutely ill, chronically ill, immunocompromised, immunosuppressed, receiving cancer chemotherapy or has anorexia nervosa.

7. The use according to any one of claims 1-6 wherein the medicament further comprises a recombinant growth hormone or a growth hormone secretagogue selected from the group consisting of GHRP-6, GHRP-1, GHRP-2, hexarelin, growth hormone releasing factor, an analog of growth hormone releasing factor, IGF-I and IGF-II.

8. The use according to any one of claims 1-6 wherein the medicament further comprises an antidepressant, a prodrug thereof or a pharmaceutically acceptable salt of said antidepressant or said prodrug.

9. The use according to claim 8 wherein said antidepressant is a norepinephrine reuptake inhibitor (NERI), selective serotonin reuptake inhibitor (SSRI), monoamine oxidase inhibitor (MAO), combined NERI/SSRI, or an atypical antidepressant, a prodrug of said antidepressant or a pharmaceutically acceptable salt of said antidepressant or said prodrug.

10. The use according to claim 9 wherein said selective serotonin reuptake inhibitor (SSRI) is citalopram, femoxetine, fluoxetine, fluvoxamine, indalpine, indeloxazine, milnacipran, paroxetine, sertraline, sertraline hydrochloride, sibutramine or zimeldine, a prodrug of said SSRI or a pharmaceutically acceptable salt of said SSRI or said prodrug.

11. The use according to any one of claims 1-6 wherein the medicament further comprises an antiemetic agent, a prodrug thereof or a pharmaceutically acceptable salt of said antiemetic or said prodrug.

12. The use of claim 11 wherein the antiemetic agent is meclizine hydrochloride, prochlorperazine, promethazine, trimethobenzamide hydrochloride or ondansetron hydrochloride.

13. The use according to any one of claims 1-6 wherein the medicament further comprises an antipsychotic agent, a prodrug thereof or a pharmaceutically acceptable salt of said antipsychotic agent or said prodrug.

14. The use of claim 13 wherein the antipsychotic agent is chlorpromazine, haloperidol, clozapine, loxapine, molindone hydrochloride, thiothixene, olanzapine, ziprasidone, ziprasidone hydrochloride, prochlorperazine, perphenazine, trifluoperazine hydrochloride or risperidone.
